(19) Europäisches Patentamt
European Patent Office
Office européen des brevets

(11) EP 0 595 150 B1

(12) **EUROPÄISCHE PATENTSCHRIFT**

(45) Veröffentlichungstag und Bekanntmachung des Hinweises auf die Patenterteilung:
**31.07.1996 Patentblatt 1996/31**

(51) Int. Cl.$^6$: **C07B 39/00**

(21) Anmeldenummer: **93116788.6**

(22) Anmeldetag: **18.10.1993**

(54) **Verfahren zur Herstellung von aromatischen Brommethyl-Verbindungen**

Process for the preparation of aromatic bromomethylcompounds

Procédé de préparation de composés aromatiques ayant des groupes bromomethyl

(84) Benannte Vertragsstaaten:
**BE CH DE ES FR GB IE IT LI**

(30) Priorität: **29.10.1992 DE 4236476**
**18.03.1993 DE 4308562**

(43) Veröffentlichungstag der Anmeldung:
**04.05.1994 Patentblatt 1994/18**

(73) Patentinhaber: **HOECHST AKTIENGESELLSCHAFT**
**65929 Frankfurt am Main (DE)**

(72) Erfinder:
- **Wagner, Adalbert, Dr.**
  **D-65795 Hattersheim (DE)**
- **Kleemann, Heinz-Werner, Dr.**
  **D-61350 Bad Homburg (DE)**
- **Regnat, Dieter, Dr.**
  **D-60529 Frankfurt am Main (DE)**
- **Kleiner, Hans-Jerg, Dr.**
  **D-61476 Kronberg/Taunus (DE)**

(56) Entgegenhaltungen:
- **SYNTHETIC COMMUNICATION Bd. 23, Nr. 5 , 1993 Seiten 671 - 679 CLAUDE VACCHER ET AL. 'Bromination of alpha-Methylstyrenes with N-Bromo- succinimide in Chlorobenzene. One-Pot and Selective Preparation of 1,3-Dibromo- 2-phenylprop-1-enes'**
- **SYNLETT Bd. 6 , Juni 1992 , STUTTGART DE Seiten 531 - 533 SUGIURA TSUNEYUKI ET AL. 'Synthesis of a Novel Serotonin-3 (5-HT3) Receptor Antagonist'**
- **JOURNAL OF THE AMERICAN CHEMICAL SOCIETY. Bd. 112, Nr. 21 , 10. Oktober 1990 , GASTON, PA US Seiten 7790 - 7791 XIAO-MIN WANG ET AL. 'A Planar and Stable Derivative of 13,14-Didehydrotriben- zo[a,c,e]cyclooctene: Synthesis and X-ray Crystal Structure of 5,6-Didehydro-1,1, 14,14-tetramethyl-10,11-methano-1H-ben- zo[5,6]cycloocta[1,2,3,4-def]fluorene'**
- **HOUBEN-WEYL 'Methoden der Organischen Chemie, Band V/4: Halogenverbindungen' 1960 , GEORG THIEME VERLAG , STUTTGART**

Bemerkungen:
Die Akte enthält technische Angaben, die nach dem Eingang der Anmeldung eingereicht wurden und die nicht in dieser Patentschrift enthalten sind.

EP 0 595 150 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.0/3.4

## Beschreibung

Brommethyl-Derivate haben sich seit langem als wertvolle Zwischenprodukte zur Herstellung von Katalysatoren und Wirkstoffen wie beispielsweise Pflanzenschutzmitteln oder Pharmaka erwiesen. Im allgemeinen werden aromatische Brommethyl-Verbindungen durch radikalische Bromierung gewonnen (siehe z.B. Römpps Chemielexikon, Bd. 1, Seite 521, 8. Aufl. (1979), Franckhsche Verlagshandlung Stuttgart und Houben-Weyl Bd V/4 (1960) Seiten 341 bis 346).

Ausgehend von aromatischen Methyl-Verbindungen lassen sich durch radikalische Bromierung mit Bromierungsagentien, wie z.B. N-Bromsuccinimid oder 1,3-Dibrom-5,5-dimethylhydantoin, gegebenenfalls unter Zusatz eines Radikalstarters, wie beispielsweise Azoisobuttersäurenitril oder Benzoylperoxid, die entsprechenden aromatischen Brommethyl-Verbindungen herstellen. Bei dieser Umsetzung finden im allgemeinen Lösungsmittel wie Tetrachlormethan Verwendung, welche eine hohe Toxizität aufweisen und die darüber hinaus leicht flüchtig sind.

Aus Houben-Weyl Bd V/4 (1960), Seite 341 letzter Absatz geht hervor, daß stark polare Lösungsmittel für eine Seitenkettenbromierung nicht geeignet sind, da N-Bromsuccinimid in stark polaren Lösungsmitteln den aromatischen Kern angreift.

In Synlett Bd 6 (1992), Seiten 531 bis 533 ist die Umsetzung von 5-Chlor-2-methyl-4-nitrobenzoesäuremethylester mittels N-Bromsuccinimids in Chlorbenzol zu 5-Chlor-4-dibrommethyl-4-nitrobenzoesäuremethylester beschrieben. Die Dibromierung und die nachfolgende Hydrolyse des ruhen bromierten Reaktionsproduktes ergibt eine Ausbeute von lediglich 51%.

Gegenstand der vorliegenden Erfindung ist ein Verfahren zur Herstellung von aromatischen Brom-Methyl-Verbindungen der allgemeinen Formel (I)

$$A(CH_2 - Br)_n \qquad (I)$$

bei dem eine aromatische Verbindung $A(CH_3)_n$ (E), welche eine oder mehrere Methylgruppen enthält, in einer radikalischen Bromierungsreaktion unter Verwendung des Lösungsmittels Chlorbenzol zum Brommethyl-Derivat der Formel (I) umgesetzt wird, wobei n eine Zahl, 1, 2 oder 3, vorzugsweise 1 oder 2, bedeutet.

$$A(CH_3)_n \xrightarrow[\text{Chlorbenzol}]{[Br]} A(CH_2 - Br)_n$$

Bei Verwendung von Chlorbenzol als Lösungsmittel kann überraschenderweise eine verbesserte Reaktionsausbeute erhalten werden. Darüberhinaus ergeben sich aufgrund der geringeren Giftigkeit des Lösungsmittels Chlorbenzol verfahrenstechnische Vereinfachungen, was insbesondere bei der Herstellung im Technikums- oder Produktionsmaßstab von Vorteil ist. Aufgrund des hohen Siedepunktes von Chlorbenzol (132°C) kann bei technischen Synthesen je nach Reaktivität des Eduktes in einem größeren Temperaturbereich eine Optimierung der Reaktionsbedingungen durchgeführt werden.

Die Erfindung bezieht sich vorzugsweise auf ein Verfahren zur Herstellung von solchen aromatischen Brommethyl-Verbindungen der allgemeinen Formel (I),

$$A(CH_2 - Br)_n \qquad (I)$$

in denen n eine ganze Zahl 1, 2 oder 3, vorzugsweise 1 oder 2, bedeutet und die Gruppierung A für einen mono-, bi-, tri-, tetra-, penta- oder hexacyclischen Aryl- oder Heteroarylrest steht, der gegebenenfalls einfach, zweifach, dreifach oder vierfach substituiert sein kann.

Die Gruppierung A kann auch durch mehrere $-CH_3$-Gruppen substituiert sein, die je nach eingesetzter Menge an Bromierungsagentien dann alle oder nur zum Teil in $CH_2$-Br-Gruppen überführt werden.

Unter Aryl ist vorzugsweise ein ($C_6$-$C_{12}$)-Arylrest, wie beispielsweise Phenyl, Naphthyl, Biphenyl oder Binaphthyl zu verstehen, insbesondere jedoch Phenyl oder Biphenyl.

Bevorzugt wird auch die zweifache Bromierung eines Binaphthylderivats, welches zwei Methylgruppen enthält.

Unter Heteroaryl werden bevorzugt ($C_1$-$C_9$)-Heteroarylgruppen verstanden, die sich von Phenyl oder Naphthyl ableiten und in welchen eine oder mehrere ICH Gruppen durch Stickstoff ersetzt sind und/oder in welchen mindestens zwei benachbarte CH-Gruppen unter Bildung eines 5-gliedrigen aromatischen Rings durch ein Schwefelatom, ein Sauerstoffatom oder eine NH-Gruppe ersetzt sind. Bevorzugte Heteroarylgruppen sind folgende Gruppierungen:
Furanyl, Thienyl, Pyrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxylinyl, Chinazolinyl, Cinnolinyl, Benzothienyl, Pyridazopyridyl, Pyridazopyrimidinyl, Pyridinothienyl sowie Imidazopyridyl.

Besonders bevorzugt sind als Heteroaromaten Benzothiophen, Pyridazopyridin, Pyridazopyrimidin, Thienyl, Benzothiazol und Pyridinothienyl.

Als Substituenten kommen im Prinzip alle üblichen Substituenten von aromatischen Systemen in Frage, die selbst nicht radikalisch bromiert sind.

Als Substituenten geeignet sind z.B. Halogen, insbesondere F, Cl, Br; $-(CH_2)_m-COOR^1$, $-CONR^2$, $-SO_2NR^2$, $-SO_2-R^3$, Phenyl, Phenyl mono- oder di-substituiert durch Halogen, -CN, $-(C_1-C_4)$-Alkyl, welches durch $COOR^1$ substituiert ist und $-(C_2-C_4)$-Alkenyl, welches durch $COOR^1$ substituiert ist.

$R^1$ bedeutet hierbei $(C_1-C_6)$-Alkyl oder Wasserstoff;
$R^2$ bedeutet

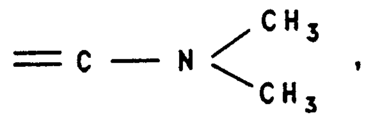

$R^3$ bedeutet $NHCOOR^1$; und
m ist eine ganze Zahl von 0 bis 4, vorzugsweise 1 oder 2.

Die bei der radikalischen Bromierung unter Verwendung des Lösungsmittels Chlorbenzol gewonnenen Brommethyl-Verbindungen werden in der Regel in so hoher Ausbeute gewonnen, daß eine weitergehende Reinigung nicht erforderlich ist. Sie können dann ohne aufwendige Trennverfahren für die weitere Synthese eingesetzt werden. Erforderlichenfalls können Brommethyl-Verbindungen mit besonderer Reinheit durch gängige Methoden, wie beispielsweise Chromatographie, Destillation oder Kristallisation erhalten werden.

Die Reaktionstemperatur bei dem erfindungsgemäßen Verfahren beträgt (bei Normaldruck) vorzugsweise zwischen Raumtemperatur und Siedetemperatur, insbesondere von 60 bis 132°C. Prinzipiell ist die Durchführung der Bromierung auch unter erhöhtem Druck durchführbar.

Als Bromierungsagentien können übliche bromhaltige Verbindungen eingesetzt werden, als besonders geeignet hat sich N-Bromsuccinimid ergeben. Die Reaktionszeit beträgt zwischen wenigen Minuten und mehreren Stunden, je nach Reaktivität der eingesetzten aromatischen Methyl-Verbindung.

Die Erfindung wird durch nachfolgende Beispiele näher erläutert:

Beispiel 1

Verfahren zur Herstellung von 3-Chlor-6-brommethylbenzo[b]thiophen-2-carbonsäuremethylester

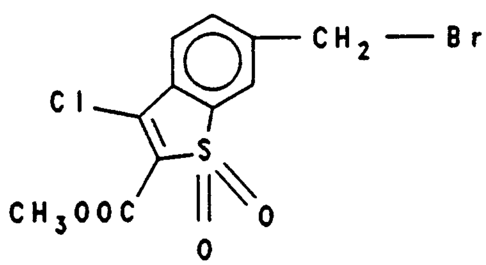

Zu 15 g (62,5 mmol) 3-Chlor-6-methylbenzo[b]thiophen-2-carbonsäuremethylester in 100 ml Chlorbenzol gibt man unter Rückfluß bei Siedetemperatur portionsweise ein Gemisch aus 11 g (62 mmol) NBS und 100 mg Benzoylperoxid. 30 Minuten nach Beendigung der Zugabe wird das Lösungsmittel verdampft. Der verbleibende Rest wird in 500 ml Ethylacetat aufgenommen und je 1 x mit 10 %iger $Na_2SO_3$-Lösung, gesättigter $Na_2CO_3$ und gesättigter NaCl-Lösung gewaschen. Nach dem Trocknen mit $MgSO_4$ wird eingeengt.

Ausbeute: 19 g (88 %);
die Titelverbindung ist gemäß Dünnschichtchromatogramm einheitlich;
$R_1$(E/H 1/4) = 0,4. Das Produkt kann ohne weitere Reinigung für Folgereaktionen verwendet werden, beispielsweise zur Synthese von Wirkstoffen.

Auch bei Durchführung des Syntheseverfahrens in deutlich größerem Maßstab, z. B. im Kilomaßstab kann eine hohe Produktqualität mit einer einfachen Verfahrensdurchführung erreicht werden.

Analog werden die Verbindungen gemäß den nachfolgenden Beispielen 2 bis 32 erhalten.

| Bsp. Nr. | $A - CH_2 - Br$ | MS ($M^+ + H$) | $R_f$ (Laufmittel) |
|---|---|---|---|
| 2 | 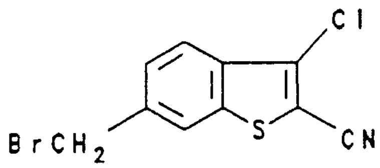 | 286 | 0,2 (E/H 1/4) |
| 3 | 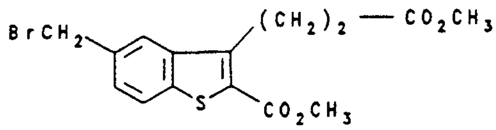 | 371 | 0,6 (E/H 1/2) |
| 4 | 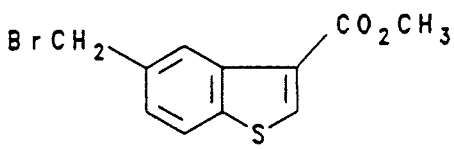 | 285 | 0,4 (E/H 1/2) |

| Bsp. Nr. | $A - CH_2 - Br$ | MS ($M^+ + H$) | $R_f$ (Laufmittel) |
|---|---|---|---|
| 5 | 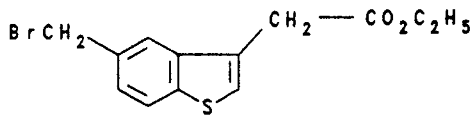 | 313 | 0,35 (E/H 1/4) |
| 6 | 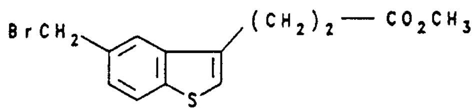 | 313 | 0,15 (E/H 4/1) |
| 7 | 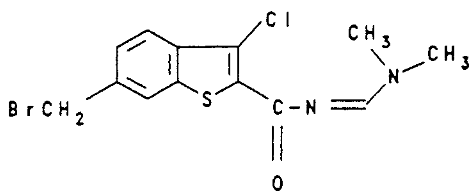 | 361 | 0,4 (E/H 1/1) |
| 8 | 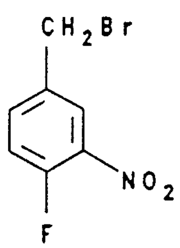 | 234 | destilliert |
| 9 | 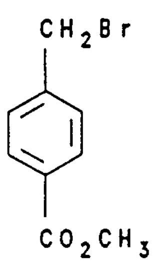 | 230 | 0,8 (E/H 2/1) |

| Bsp. Nr. | A - $CH_2$ - Br | MS ($M^+$ + H) | $R_f$ (Lauf-mittel) |
|---|---|---|---|
| 10 | 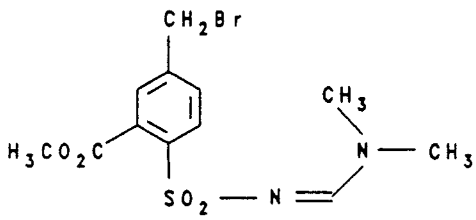 | 363 | 0,35 (E) |
| 11 | 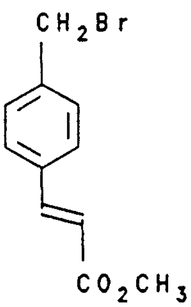 | 253 | 0,5 (E/H 1/4) |
| 12 | 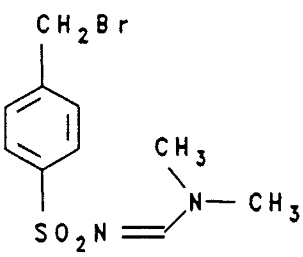 | 305 | 0,3 (E/H 1/1) |
| 13 | 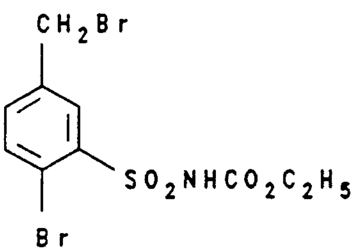 | 400 | 0,4 (E/H/$CH_3CO_2$H 1/1/+1%) |

| Bsp. Nr. | $A - CH_2 - Br$ | MS ($M^+ + H$) | $R_f$ (Laufmittel) |
|---|---|---|---|
| 14 | 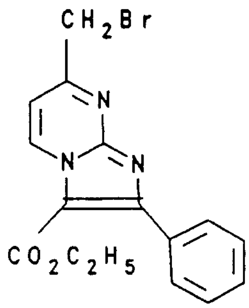 | 360 | 0,3 (E/H 1/1) |
| 15 | 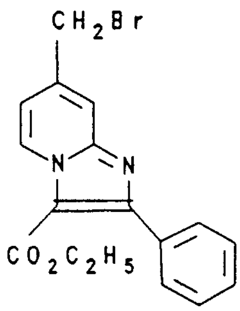 | 359 | 0,5 (E/H 1/1) |
| 16 | 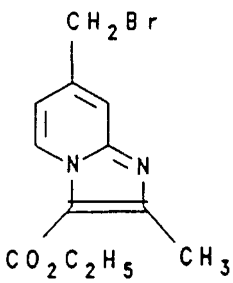 | 297 | 0,4 (E/H 1/1) |

| Bsp. Nr. | $A - CH_2 - Br$ | MS ($M^+ + H$) | $R_f$ (Laufmittel) |
|---|---|---|---|
| 17 | 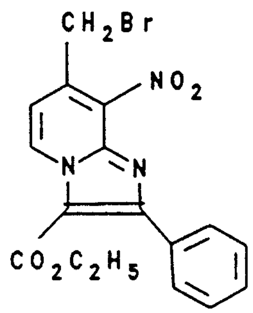 | 404 | 0,6 (E/H 1/1) |
| 18 | 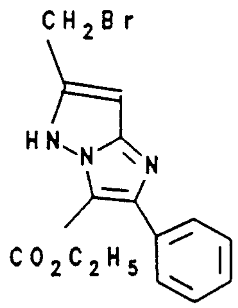 | 348 | 0,6 (E/H 1/1) |
| 19 | 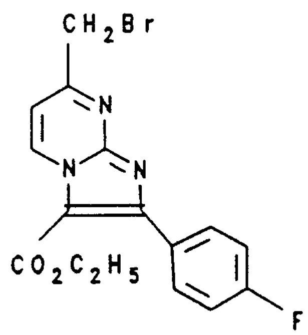 | 378 | 0,35 (E/H 1/1) |

| Bsp. Nr. | A - $CH_2$ - Br | MS ($M^+$+H) | $R_f$ (Laufmittel) |
|---|---|---|---|
| 20 | 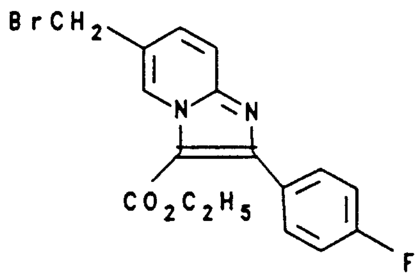 | 377 | 0,3 (E/H 1/1) |
| 21 | 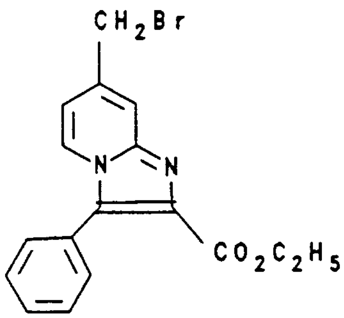 | 359 | 0,25 (E/H 1/1) |
| 22 | 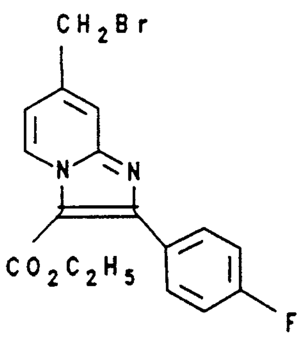 | 377 | 0,3 (E/H 1/1) |

| Bsp. Nr. | $A - CH_2 - Br$ | MS $(M^+ + H)$ | $R_f$ (Laufmittel) |
|---|---|---|---|
| 23 | 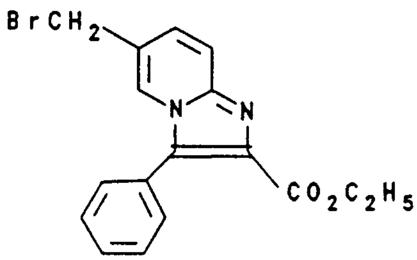 | 359 | 0,3 (E/H 4/1) |
| 24 | 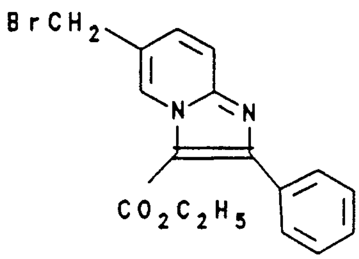 | 359 | 0,3 (H/E 1/1) |
| 25 | 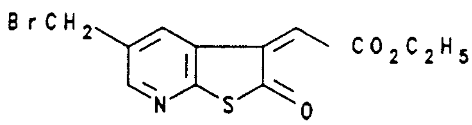 | 326 | 0,3 (H/E 1/1) |
| 26 | 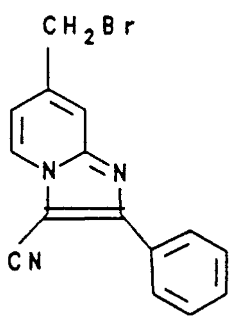 | 312 | 0,4 (E/H 1/1) |

| Bsp. Nr. | $A - CH_2 - Br$ | MS ($M^+ + H$) | $R_f$ (Laufmittel) |
|---|---|---|---|
| 27 | 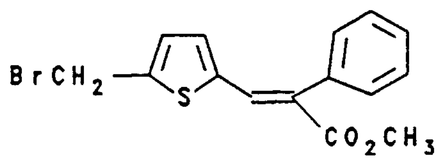 | 337 | 0,4 (E/H 1/1) |
| 28 | 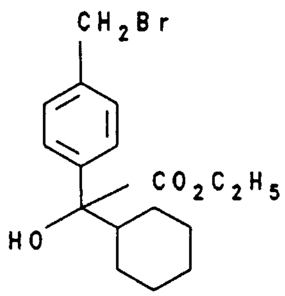 | 356 | 0,15 (DIP/H 1/10) |
| 29 | 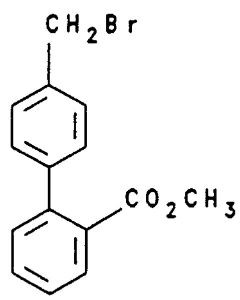 | 306 | 0,15 (DIP/H 1/10) |
| 30 | 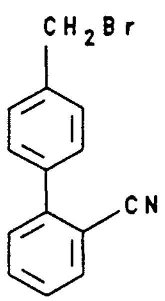 | 272 | 0,2 (DIP/H 1/1) |

| Bsp. Nr. | A - $CH_2$ - Br | MS ($M^+$ +H) | $R_f$ (Lauf- mittel) |
|---|---|---|---|
| 31 | 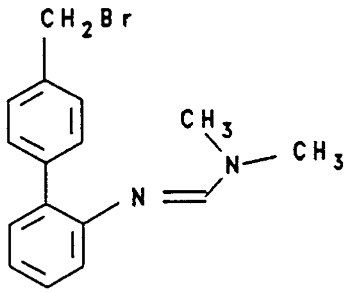 | 317 | 0,4 (E/H 1/1) |
| 32 | 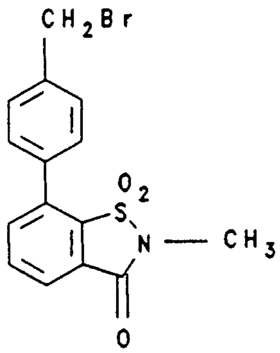 | 366 | 0,4 (DIP) |

| Abkürzungen: | | | |
|---|---|---|---|
| | H | = | n-Heptan |
| | E | = | Ethylacetat |
| | DIP | = | Diisopropylether |
| | NBS | = | N-Bromsuccinimid |

Beispiel 33

Als Beispiel für eine mehrfache Methylgruppen-Bromierung wird das Verfahren zur Herstellung von 2,2'-Bis-(brommethyl)-1,1'-binaphthyl vorgestellt:

Zu 8,5 g (30 mmol) 2,2-Dimethyl-1,1'-binaphthyl in 100 ml Chlorbenzol gibt man unter Rückfluß bei Siedetemperatur portionsweise eine Gemisch aus 10,7 g (60 mmol) N-Bromsuccinimid und 100 mg Benzoylperoxid. Nach beendeter Zugabe wird noch 1 Stunde bei Siedetemperatur gerührt und das Lösungsmittel verdampft. Der Rückstand wird in 50 ml Ethylacetat aufgenommen und je 1 x mit 10 %iger $Na_2SO_3$-Lösung, gesättigter $Na_2CO_3$ und gesättigter NaCl-Lösung gewaschen. Nach dem Trocknen mit $MgSO_4$ wird eingeengt.

Man erhält 13,2 g (100 %) gelbes Öl.

Aus Toluol kristallisieren 8,6 g farblose Kristalle mit Schmp. 147-149°C.

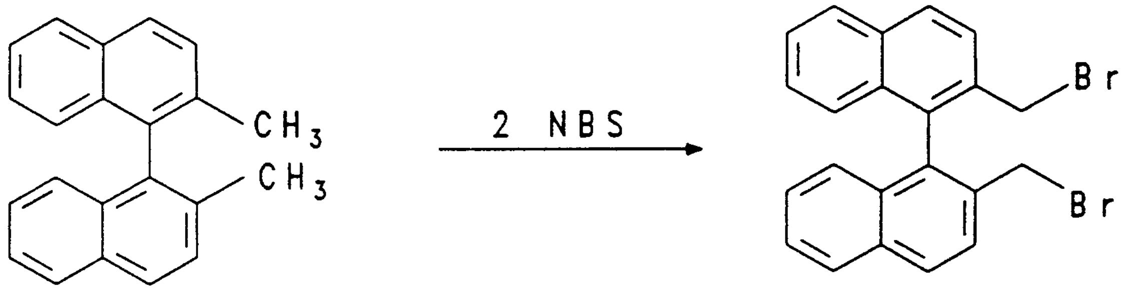


## Patentansprüche

1. Verfahren zur Herstellung von aromatischen Brommethyl-Verbindungen, bei dem ausgehend von einer aromatischen Methyl-Verbindung durch radikalische Bromierung im Lösungsmittel Chlorbenzol die Brommethyl-Verbindung erhalten wird.

2. Verfahren gemäß Anspruch 1, dadurch gekennzeichnet, daß man ausgehend von einer Verbindung (E)

$$A(CH_3)_n \qquad (E)$$

eine Verbindung der Formel (I) herstellt,

$$A(CH_2 - Br)_n \qquad (I)$$

wobei A für einen aromatischen Rest steht, der gegebenenfalls substituiert ist, und n eine ganze Zahl 1, 2 oder 3 bedeutet.

3. Verfahren gemäß Anspruch 2, dadurch gekennzeichnet, daß A für einen mono-, bi- oder tricyclischen Aryl- oder Heteroarylrest steht, der gegebenenfalls einfach, zweifach, dreifach oder vierfach substituiert sein kann.

4. Verfahren gemäß Anspruch 3, dadurch gekennzeichnet, daß A für einen $(C_6-C_{12})$-Arylrest oder für einen $(C_1-C_9)$-Heteroarylrest steht, wobei diese durch einen, zwei, drei oder vier Substituenten aus der Gruppe: Halogen, -$(CH_2)_m$-$COOR^1$, -$CONR^2$, -$SO_2NR^2$, -$SO_2$-$R^3$, -Phenyl, -Phenyl substituiert durch Halogen, -CN, -$(C_1-C_4)$-Alkyl, weches durch $COOR^1$ substituiert ist und -$(C_2-C_4)$-Alkenyl, welches durch $COOR^1$ substituiert ist, substituiert sein kann,
wobei

$R^1$ $(C_1-C_6)$-Alkyl oder Wasserstoff bedeutet,
$R^2$

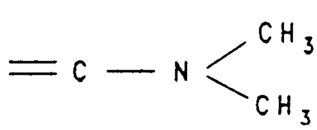
,

bedeutet, bedeutet,
$R^3$ $NHCOOR^1$ bedeutet, und
m eine ganze Zahl von 0 bis 4 ist.

5. Verfahren gemäß Anspruch 3, dadurch gekennzeichnet, daß A für ein Binaphthyl-System steht und n gleich 2 ist.

**6.** Verfahren gemäß Anspruch 1, dadurch gekennzeichnet, daß die radikalische Bromierung unter Verwendung von N-Bromsuccinimid durchgeführt wird.

**7.** Vefahren gemäß Anspruch 5, dadurch gekennzeichnet, daß die Bromierung unter Einsatz eines Radikalbildners erfolgt.

## Claims

**1.** A process for the preparation of aromatic bromomethyl compounds, which comprises using an aromatic methyl compound as the starting material to obtain the bromomethyl compound by free-radical bromination in the solvent chlorobenzene.

**2.** The process as claimed in claim 1, wherein a compound (E)

$$A(CH_3)_n \qquad (E)$$

is used as the starting material to prepare a compound of formula (I)

$$A(CH_2\text{-}Br)_n \qquad (I),$$

A being an optionally substituted aromatic radical and n being the integer 1, 2 or 3.

**3.** The process as claimed in claim 2, wherein A is a mono-, bi- or tricyclic aryl or heteroaryl radical optionally substituted by one, two, three or four substituents.

**4.** The process as claimed in claim 3, wherein A is a $(C_6\text{-}C_{12})$-aryl radical or a $(C_1\text{-}C_9)$-heteroaryl radical, it being possible for said radicals to be substituted by one, two, three or four substituents selected from the group consisting of halogen, $-(CH_2)_m\text{-}COOR^1$, $-CONR^2$, $-SO_2NR^2$, $-SO_2\text{-}R^3$, phenyl, phenyl substituted by halogen, -CN, $-(C_1\text{-}C_4)$-alkyl substituted by $COOR^1$ and $-(C_2\text{-}C_4)$-alkenyl substituted by $COOR^1$,

$R^1$ being $(C_1\text{-}C_6)$-alkyl or hydrogen,
$R^2$ being

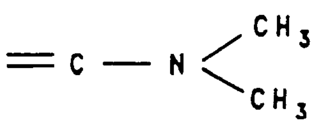 ,

$R^3$ being $NHCOOR^1$ and
m being an integer from 0 to 4.

**5.** The process as claimed in claim 3, wherein A is a binaphthyl system and n is equal to 2.

**6.** The process as claimed in claim 1, wherein the free-radical bromination is carried out using N-bromosuccinimide.

**7.** The process as claimed in claim 5, wherein the bromination is carried out using a free-radical initiator.

## Revendications

**1.** Procédé pour la préparation de composés de bromométhyle aromatiques dans lequel, en partant d'un composé méthylique aromatique, on obtient par bromation radicalaire dans le solvant chlorobenzène le composé de bromométhyle.

**2.** Procédé selon la revendication 1, caractérisé en ce que en partant d'un composé (E)

$$A(CH_3)_n \qquad (E)$$

on prépare un composé de formule (I)

$$A(CH_2 - Br)_n \qquad (I)$$

dans laquelle A est le radical aromatique, qui est éventuellement substitué, et n vaut un nombre entier 1, 2 ou 3.

**3.** Procédé selon la revendication 1, caractérisé en ce que A représente un radical aryle ou hétéroaryle mono-, bi- ou tricyclique, qui peut être éventuellement substitué une fois, deux fois, trois frois ou quatre fois.

**4.** Procédé selon la revendication 3, caractérisé en ce que A représente un radical aryle en $C_6$-$C_{12}$ ou un radical hétéroaryle en $C_1$-$C_9$ ceux-ci pouvant être substitués par un, deux, trois ou quatre substituants pris dans le groupe : halogène, $-(CH_2)_m$-$COOR^1$, $-CONR^2$, $-SO_2NR^2$, $-SO_2$-$R^3$, phényle, phényle substitué par halogène, -CN, alkyle en $C_1$-$C_4$, qui est substitué par $COOR^1$ et alcényle en $C_2$-$C_4$ qui est substitué par $COOR^1$,

$R^1$ représente alkyle en $C_1$-$C_6$ ou l'hydrogène ;

$R^2$ représente

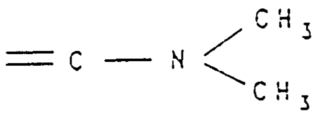

,

$R^3$ représente $NHCOOR^1$ ; et

m est un nombre entier de 0 à 4.

**5.** Procédé selon la revendication 3, caractérisé en ce que A représente un système binaphtyle et n est égal à 2.

**6.** Procédé selon la revendication 3, caractérisé en ce que la bromation radicalaire est mise en oeuvre en utilisant le N-bromosuccinimide.

**7.** Procédé selon la revendication 5, caractérisé en ce qu'on effectue la bromation en utilisant un formateur de radicaux.